# EUROPEAN PATENT SPECIFICATION

(11) **EP 1 244 435 B1**
(45) Date of publication and mention of the grant of the patent: **14.11.2007**
(21) Application number: 00989636.6
(22) Date of filing: 28.12.2000
(51) Int. Cl.: A61K 31/00

(54) **POLYHYDROXYLATED AROMATIC COMPOUNDS FOR THE TREATMENT OF AMYLOIDOSIS AND ALPHA-SYNUCLEIN FIBRIL DISEASES**
POLYHYDROXYLIERTE AROMATISCHEN VERBINDUNGEN FÜR DIE BEHANDLUNG VON AMYLOIDOSIS UND ALPHA-SYNUCLEIN FIBRIL KRANKHEITEN
COMPOSES AROMATIQUES POLYHYDROXYLES UTILISES DANS LE TRAITEMENT DE L'AMYLOIDOSE ET DE MALADIES CARACTERISEES PAR LA FORMATION DE FIBRES DE ALPHA -SYNUCLEINE

(30) Priority: 30.12.1999 US 173958 P; 26.12.2000 US 748748
(43) Date of publication of application: 02.10.2002
(60) Divisional application: 07008782.0
(73) Proprietor: Proteotech Inc., Kirkland, WA 98034 (US)
(72) Inventor: CASTILLO, Gerardo, M., Seattle, WA 98178 (US); CHOI, Paula, Y., Bothell, WA 98011 (US); SNOW, Alan, D., Lynnwood, WA 98037 (US)
(74) Representative: Jones Day
(86) International application number: PCT/US2000/035715
(87) International publication number: WO 2001/049281

(56) References cited:
- WO-A-98/09653
- WO-A-98/24646
- JP-A- 1 151 514
- US-A- 5 767 126
- C. HERTEL ET. AL.: "Inhibition of the electrostatic interaction between beta-amyloid peptide and membranes prevents beta-amyloid toxicity." PROCEEDINGS OF THE NATIONAL ACADEMY OF SCIENCES OF THE USA, vol. 94, no. 8, August 1997 (1997-08), pages 9412-6, XP001026042
- H. NIWANO ET. AL.: "Inhibitory action of amyloid precursor protein against human Hageman Factor (Factor XII)." JOURNAL OF LABORATORY AND CLINICAL MEDICINE, vol. 125, no. 2, 1995, pages 251-6, XP000933861

## Description

### BACKGROUND OF THE INVENTION

### FIELD OF THE INVENTION

This invention relates to the use of certain polyhydroxylated aromatic compounds, and compositions containing them, for the treatment of amyloidosis, especially Alzheimer's disease.

### DESCRIPTION OF THE RELATED ART

### Amyloid and amyloidosis

Amyloid is a generic term referring to a group of diverse but specific extracellular protein deposits which all have common morphological properties, staining characteristics, and X-ray diffraction spectra. Regardless of the nature of the amyloid protein deposited all amyloids have the following characteristics: 1) showing an amorphous appearance at the light microscopic level, appearing eosinophilic using hematoxylin and eosin stains; 2) staining with Congo red and demonstrating a red/green birefringence as viewed under polarized light (Puchtler et al., J. Histochem. Cytochem. 10:355-364, 1962), 3) containing a predominant beta-pleated sheet secondary structure, and 4) ultrastructurally consisting of non-branching fibrils of indefinite length and with a diameter of 7-10 nm.

Amyloidoses today are classified according to the specific amyloid protein deposited. The amyloids include, but are not limited to, the amyloid associated with Alzheimer's disease, Down's syndrome and hereditary cerebral hemorrhage with amyloidosis of the Dutch type (where the specific amyloid is referred to as beta-amyloid protein or Aβ), the amyloid associated with chronic inflammation, various forms of malignancy and familial Mediterranean fever (where the specific amyloid is referred to as AA amyloid or inflammation-associated amyloid), the amyloid associated with multiple myeloma and other B-cell dyscrasias (where the specific amyloid is referred to as AL amyloid), the amyloid associated with type II diabetes (where the specific amyloid is referred to as amylin or islet amyloid), the amyloid associated with the prion diseases including Creutzfeldt-Jakob disease, Gerstmann-Straussler syndrome, kuru, and scrapie (where the specific amyloid is referred to as PrP amyloid), the amyloid associated with long-term hemodialysis and carpal tunnel syndrome (where the specific amyloid is referred to as beta₂-microglobulin amyloid), the amyloid associated with senile cardiac amyloid and familial amyloidotic polyneuropathy (where the specific amyloid is referred to as prealbumin or transthyretin amyloid), and the amyloid associated with endocrine tumors such as medullary carcinoma of the thyroid (where the specific amyloid is referred to as variants of procalcitonin).

Although amyloid deposits in clinical conditions share common physical properties relating to the presence of a beta-pleated sheet conformation, it is now clear that many different chemical types exist and additional ones are likely to be described in the future. It is currently thought that there are several common pathogenetic mechanisms that may be operating in amyloidosis in general. In many cases, a circulating precursor protein may result from overproduction of either intact or aberrant molecules (for example, in plasma cell dyscrasias), reduced degradation or excretion (serum amyloid A in some secondary amyloid syndromes and beta₂-microglobulin in long-term hemodialysis), or genetic abnormalities associated with variant proteins (for example, familial amyloidotic polyneuropathy). Proteolysis of a larger protein precursor molecule occurs in many types of amyloidosis, resulting in the production of lower molecular weight fragments that polymerize and assume a beta-pleated sheet conformation as tissue deposits, usually in an extracellular location. The precise mechanisms involved and the aberrant causes leading to changes in proteolytic processing and/or translational modification are not known in most amyloids.

Systemic amyloids which include the amyloid associated with chronic inflammation, various forms of malignancy and familial Mediterranean fever (i.e. AA amyloid or inflammation-associated amyloidosis) (Benson and Cohen, Arth. Rheum. 22:36-42, 1979; Kamei et al, Acta Path. Jpn. 32:123-133, 1982; McAdam et al., Lancet 2:572-573, 1975; Metaxas, Kidney Int. 20:676-685, 1981), and the amyloid associated with multiple myeloma and other B-cell dyscrasias (i.e. AL amyloid) (Harada et al., J. Histochem. Cytochem. 19:1-15, 1971), as examples, are known to involve amyloid deposition in a variety of different organs and tissues generally lying outside the central nervous system. Amyloid deposition in these diseases may occur, for example, in liver, heart, spleen, gastrointestinal tract, kidney, skin, and/or lungs (Johnson et al, N. Engl. J. Med. 321:513-518,1989). For most of these amyloidoses, there is no apparent cure or effective treatment and the consequences of amyloid deposition can be detrimental to the patient. For example, amyloid deposition in the kidney may lead to renal failure, whereas amyloid deposition in the heart may lead to heart failure. For these patients, amyloid accumulation in systemic organs leads to eventual death generally within 3-5 years. Other amyloidoses may affect a single organ or tissue such as observed with the Aβ amyloid deposits found in the brains of patients with Alzheimer's disease and Down's syndrome: the PrP amyloid deposits found in the brains of patients with Creutzfeldt-Jakob disease, Gerstmann-Straussler syndrome, and kuru; the islet amyloid (amylin) deposits found in the islets of Langerhans in the pancreas of 90% of patients with type II diabetes (Johnson et al, N. Engl. J. Med. 321:513-518, 1989; Lab. Invest. 66:522 535, 1992); the beta₂-microglobulin amyloid deposits in the medial nerve leading to carpal tunnel syndrome as observed in patients undergoing long-term hemodialysis (Geyjo et al, Biochem. Biophys. Res. Comm. 129:701-706, 1985; Kidney Int. 30:385-390, 1986); the prealbumin/transthyretin amyloid observed in the hearts of patients with senile cardiac amyloid; and the prealbumin/transthyretin amyloid observed in peripheral nerves of patients who have familial amyloidotic polyneuropathy (Skinner and Cohen, Biochem. Biophys. Res. Comm. 99:1326-1332, 1981; Saraiva et al, J. Lab. Clin. Med. 102:590-603, 1983; J. Clin. Invest. 74:104-119, 1984; Tawara et al, J. Lab. Clin. Med. 98:811-822, 1989).

### Alzheimer's disease and the aging population

Alzheimer's disease is a leading cause of dementia in the elderly, affecting 5-10% of the population over the age of 65 years (A Guide to Understanding Alzheimer's Disease and Related Disorders, Jorm, ed., New York University Press, New York, 1987). In Alzheimer's disease, the parts of the brain essential for cognitive processes such as memory, attention, language, and reasoning degenerate, robbing victims of much that makes us human, including independence. In some inherited forms of Alzheimer's disease, onset is in middle age, but more commonly, symptoms appear from the mid-60's onward. Alzheimer's disease today affects 4-5 million Americans, with slightly more than half of these people receiving care at home, while the others are in many different health care institutions. The prevalence of Alzheimer's disease and other dementias doubles every 5 years beyond the age of 65, and recent studies indicate that nearly 50% of all people age 85 and older have symptoms of Alzheimer's disease (*1999 Progress Report on Alzheimer's Disease,* National Institute on Aging/National Institute of Health). 13% (33 million people) of the total population of the United States are age 65 and older, and this percentage will climb to 20% by the year 2025 (*1999 Progress Report on Alzheimer's Disease).*

Alzheimer's disease also puts a heavy economic burden on society. A recent study estimated that the cost of caring for one Alzheimer's disease patient with severe cognitive impairments at home or in a nursing home, is more than $47,000 per year (A *Guide to Understanding Alzheimer's Disease and Related Disorders*). For a disease that can span from 2 to 20 years, the overall cost of Alzheimer's disease to families and to society is staggering. The annual economic toll of Alzheimer's disease in the United States in terms of health care expenses and lost wages of both patients and their caregivers is estimated at $80 to $100 billion (*1999 Progress Report on Alzheimer's Disease*).

Tacrine hydrochloride ("Cognex"), the first FDA approved drug for Alzheimer's disease, is a acetylcholinesterase inhibitor (Cutler and Sramek, N. Engl. J. Med. 328:808 810, 1993). However, this drug has showed limited success in producing cognitive improvement in Alzheimer's disease patients and initially had major side effects such as liver toxicity. The second more recently FDA approved drug, donepezil ("Aricept"), which is also an acetylcholinesterase inhibitor, is more effective than tacrine, by demonstrating slight cognitive improvement in Alzheimer's disease patients (Barner and Gray, Ann. Pharmacotherapy 32:70-77, 1998; Rogers and Friedhoff, Eur. Neuropsych. 8:67-75, 1998), but is not believed to be a cure. Therefore, it is clear that there is a need for more effective treatments for Alzheimer's disease patients.

### Amyloid as a therapeutic target for Alzheimer's disease

Alzheimer's disease is characterized by the deposition and accumulation of a 39-43 amino acid peptide termed the beta-amyloid protein, Aβ or β/A4 (Glenner and Wong, Biochem. Biophys. Res. Comm. 120:885-890, 1984; Masters et al., Proc. Natl. Acad. Sci. USA 82:4245-4249, 1985; Husby et al., Bull. WHO 71:105-108, 1993). Aβ is derived by protease cleavage from larger precursor proteins termed beta-amyloid precursor proteins (or βPPs) of which there are several alternatively spliced variants. The most abundant forms of the βPPs include proteins consisting of 695, 751 and 770 amino acids (Tanzi et al., Nature 331:528-530, 1988; Kitaguchi et al., Nature 331:530-532, 1988; Ponte et al., Nature 331:525-527, 1988).

The small Aβ peptide is a major component which makes up the amyloid deposits of "plaques" in the brains of patients with Alzheimer's disease. In addition, Alzheimer's disease is characterized by the presence of numerous neurofibrillary "tangles", consisting of paired helical filaments which abnormally accumulate in the neuronal cytoplasm (Grundke-Iqbal et al., Proc. Natl. Acad. Sci. USA 83:4913-4917, 1986; Kosik et al., Proc. Natl. Acad. Sci. USA 83:4044-4048, 1986; Lee et al., Science 251:675-678, 1991.). The pathological hallmark of Alzheimer's disease is therefore the presence of "plaques" and "tangles", with amyloid being deposited in the central core of the plaques. The other major type of lesion found in the Alzheimer's disease brain is the accumulation of amyloid in the walls of blood vessels, both within the brain parenchyma and in the walls of meningeal vessels which lie outside the brain. The amyloid deposits localized to the walls of blood vessels are referred to as cerebrovascular amyloid or congophilic angiopathy (Mandybur, J. Neuropath. Exp. Neurol. 45:79-90, 1986; Pardridge et al., J. Neurochem. 49:1394-1401, 1987).

For many years there has been an ongoing scientific debate as to the importance of "amyloid" in Alzheimer's disease, and whether the "plaques" and "tangles" characteristic of this disease were a cause or merely a consequence of the disease. Within the last few years, studies now indicate that amyloid is indeed a causative factor for Alzheimer's disease and should not be regarded as merely an innocent bystander. The Alzheimer's Aβ protein in cell culture has been shown to cause degeneration of nerve cells within short periods of time (Pike et al., Br. Res. 563:311-314, 1991; J. Neurochem. 64:253-265, 1995). Studies suggest that it is the fibrillar structure (consisting of a predominant beta-pleated sheet secondary structure), characteristic of all amyloids, that is responsible for the neurotoxic effects. Aβ has also been found to be neurotoxic in slice cultures of hippocampus (Harrigan et al., Neurobiol. Aging 16:779-789, 1995) and induces nerve cell death in transgenic mice (Games et al., Nature 373:523-527, 1995; Hsiao et al., Science 274:99-102, 1996). Injection of the Alzheimer's Aβ into rat brain also causes memory impairment and neuronal dysfunction (Flood et al., Proc. Natl. Acad. Sci. USA 88:3363-3366, 1991; Br. Res. 663:271-276, 1994).

Probably, the most convincing evidence that Aβ amyloid is directly involved in the pathogenesis of Alzheimer's disease comes from genetic studies. It has been discovered that the production of Aβ can result from mutations in the gene encoding, its precursor, beta amyloid precursor protein (Van Broeckhoven et al., Science 248:1120-1122, 1990; Murrell et al., Science 254:97-99, 1991; Haass et al., Nature Med. 1:1291-1296, 1995). The identification of mutations in the beta-amyloid precursor protein gene which causes early onset familial Alzheimer's disease is the strongest argument that amyloid is central to the pathogenetic process underlying this disease. Four reported disease-causing mutations have now been discovered which demonstrate the importance of Aβ in causing familial Alzheimer's disease (reviewed in Hardy, Nature Genet. 1:233-234, 1992). All of these studies suggest that providing a drug to reduce, eliminate or prevent fibrillar Aβ formation, deposition, accumulation and/or persistence in the brains of human patients will serve as an effective therapeutic.

Discovery and identification of new compounds or agents as potential therapeutic agents to arrest amyloid deposition, accumulation and/or persistence that occurs in Alzheimer's disease and other amyloidoses are desperately sought.

### Parkinson's Disease and α-Synuclein Fibril Formation

Parkinson's disease is a neurodegenerative disorder that is pathologically characterized by the presence of intracytoplasmic Lewy bodies (Lewy in Handbuch der Neurologie, M. Lewandowski, ed., Springer, Berlin, pp. 920-933, 1912; Pollanen et al., J. Neuropath. Exp. Neurol. 52:183-191, 1993), the major components of which are filaments consisting of α-synuclein (Spillantini et al., Proc. Natl. Acad. Sci. USA 95:6469-6473, 1998; Arai et al., Neurosc. Lett. 259:83-86, 1999), an 140-amino acid protein (Ueda et al., Proc. Natl. Acad. Sci. USA 90:11282-11286, 1993). Two dominant mutations in α-synuclein causing familial early onset Parkinson's disease have been described suggesting that Lewy bodies contribute mechanistically to the degeneration of neurons in Parkinson's disease (Polymeropoulos et al., Science 276:2045-2047, 1997; Kruger et al., Nature Genet. 18:106-108, 1998). Recently, *in vitro* studies have demonstrated that recombinant α-synuclein can indeed form Lewy body-like fibrils (Conway et al., Nature Med. 4:1318-1320, 1998; Hashimoto et al., Brain Res. 799:301-306, 1998; Nahri et al., J. Biol. Chem. 274:9843-9846, 1999). Most importantly. both Parkinson's disease-linked α-synuclein mutations accelerate this aggregation process which suggests that such *in vitro* studies may have relevance for Parkinson's disease pathogenesis. α-Synuclein aggregation and fibril formation fulfills of the criteria of a nucleation-dependent polymerization process (Wood et al., J. Biol. Chem. 274:19509-19512, 1999). In this regard α-synuclein fibril formation resembles that of Alzheimer's beta-amyloid protein (Aβ) fibrils. α-Synuclein recombinant protein, and non-amyloid component (known as NAC-P), which is a 35-amino acid peptide fragment of α-synuclein, both have the ability to form fibrils when incubated at 37°C, and are positive with amyloid stains such as Congo red (demonstrating a red/green birefringence when viewed under polarized light) and Thioflavin S (demonstrating positive fluorescence) (Hashimoto et al., Brain Res. 799:301-306, 1998; Ueda et al., Proc. Natl. Acad. Sci. USA 90:11282-11286, 1993).

Parkinson's disease α-synuclein fibrils, like the Aβ fibrils of Alzheimer's disease, also consist of a predominant beta-pleated sheet structure. We believe, therefore, that compounds found to inhibit Alzheimer's disease Aβ amyloid fibril formation can also be anticipated to be effective in the inhibition of α-synuclein fibril formation. These compounds would therefore also serve as therapeutics for Parkinson's disease, in addition to having efficacy as a therapeutic for Alzheimer's disease and other amyloid disorders.

### SUMMARY OF THE INVENTION

This invention provides a method of treating amyloidosis in a mammal suffering therefrom, comprising administration to the mammal of a therapeutically effective amount of an isolated pure compound selected from the group consisting of the compounds of formula A and formula B: 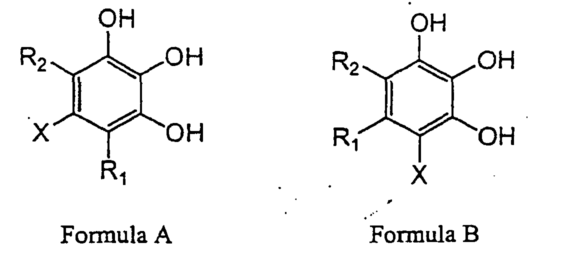 where:
R₁ and R₂ are independently selected from the group consisting of hydrogen; C₁₋₆ alkyl, C₁₋₆ alkoxy, and C₁₋₆ alkylthio (in each of which the alkyl group is optionally substituted with 1 to 5 halogen atoms); and halo;
X is selected from hydrogen and the group consisting of C₁₋₂₂ alkyl optionally substituted with 1 to 5 moieties selected from the group consisting of hydroxy and amino.
And the group of compounds consisting of 1,2,4-benzenetriol, carbidopa, deoxyepinephrine, dioxethedrine, dopa, dopamine, droxidopa,
and the pharmaceutically acceptable salts thereof.

In preferred embodiments of this first aspect, only one such compound is administered; the mammal is a human; and the amyloidosis is selected from the group consisting of Alzheimer's disease, Down's syndrome, hereditary cerebral hemorrhage with amyloidosis of the Dutch type, the amyloidosis of chronic inflammation, the amyloidosis of malignancy, familial Mediterranean fever, multiple myeloma, B-cell dyscrasias, type II diabetes, the prion diseases, Creutzfeldt-Jakob disease, Gerstmann-Straussler syndrome, kuru, scrapie, the amyloidosis associated with long-term hemodialysis, the amyloidosis associated with carpal tunnel syndrome, senile cardiac amyloidosis, familial amyloidotic polyneuropathy, and the amyloidosis associated with endocrine tumors, and especially is Alzheimer's disease.

In one embodiment a drug product for the treatment of amyloidosis in a mammal suffering therefrom is provided, comprising a container labeled or accompanied by a label indicating that the drug product is for the treatment of amyloidosis, the container containing one or more dosage units each comprising at least one pharmaceutically acceptable excipient and, as an active ingredient, an isolated pure compound selected from those used in the method of the first aspect of this invention.

Preferably, the drug product contains only one such compound, the mammal is a human; and the amyloidosis is selected from the group consisting of Alzheimer's disease, Down's syndrome, hereditary cerebral hemorrhage with amyloidosis of the Dutch type, the amyloidosis of chronic inflammation, the amyloidosis of malignancy, familial Mediterranean fever, multiple myeloma, B-cell dyscrasias, type II diabetes, the prion diseases, Creutzfeldt-Jakob disease, Gerstmann-Straussler syndrome, kuru, scrapie, the amyloidosis associated with long-term hemodialysis, the amyloidosis associated with carpal tunnel syndrome, senile cardiac amyloidosis, familial amyloidotic polyneuropathy, and the amyloidosis associated with endocrine tumors, and especially is Alzheimer's disease.

The compounds can also be used in a method of treating a disease characterized by α-synuclein fibril formation in a mammal suffering therefrom.

In one embodiment, only one such compound is administered; the mammal is a human; and the disease is Lewy body disease or Parkinson's disease, especially Parkinson's disease.

In another aspect a drug product is provided for the treatment of a disease characterized by α-synuclein fibril formation in a mammal suffering therefrom, comprising a container labeled or accompanied by a label indicating that the drug product is for the treatment of a disease characterized by α-synuclein fibril formation, the container containing one or more dosage units each comprising at least one pharmaceutically acceptable excipient and, as an active ingredient, an isolated pure compound selected from those used in the method of the third aspect of this invention.

Preferably, the drug product contains only one such compound, the mammal is a human; and the disease is Lewy body disease or Parkinson's disease, especially Parkinson's disease.

### DETAILED DESCRIPTION OF THE PREFERRED EMBODIMENTS

### Definitions

"Alkyl" means a linear hydrocarbyl group having from one to the number of carbon atoms specified, or a branched or cyclic hydrocarbyl group having from three to the number of carbon atoms specified. "Alkyl" in this application is given a broader meaning than is conventional in organic chemistry and includes both saturated groups (those conventionally known as alkyl groups), monounsaturated groups (such as those conventionally known as alkenyl and alkynyl groups), and polyunsaturated groups, except that the terms does not include groups containing aromatic moieties, as the term "aromatic" is conventionally used. Exemplary C₁₋₆ alkyl groups include methyl, ethyl, isopropyl, cyclopropyl, *tert*-butyl, cyclopropylmethyl, and hexyl.

An "aromatic" group is a cyclic (monocyclic, condensed bicyclic, or linked bicyclic) group having from 5 to 12 ring carbon atoms, and sufficient ring unsaturation that the group is "aromatic" as that term is conventionally used. Exemplary aromatic groups include phenyl, naphthyl, and biphenylyl. A "heteroaromatic" group is an "aromatic" group as just defined in which from 1 to 4 of the ring carbon atoms have been replace by O, S, or NR (where R is hydrogen or C₁₋₆ alkyl). Exemplary heteroaromatic groups include pyrrolyl, furanyl, thiophenyl, benzofuranyl, indolyl, and the like. Such aromatic and heteroaromatic groups may optionally be substituted with 1 or more, especially 1 to 3, non-interfering substituents.

An "isolated pure compound" is a compound in isolated purified form such as is conventional for active ingredients in the pharmaceutical industry, and specifically excludes the compound when found as a component in a mixture such as within a plant or part thereof, or an extract or decoction of such plant or part, even when such mixtures are partially purified to limit the number of components present therein. However, treatment with an "isolated pure compound" is not limited to treatment with the compound alone but also includes treatment with the compound when present in a pharmaceutical composition of the type conventional in pharmaceutical practice, i.e. including one or more pharmaceutical excipients; however it specifically excludes treatment with the compound when the compound is found as a component in a mixture such as within a plant or part thereof, or an extract or decoction of such plant or part, even when such mixtures are partially purified to limit the number of components present therein.

"Mammal" includes humans and non-human mammals, such as companion animals (cats, dogs, and the like) and farm animals (cattle, horses, sheep, goats, swine, and the like).

Suitable non-interfering substituents include halogen and C₁₋₆ alkyl and C₁₋₆ alkoxy, each optionally substituted with up to 5 halogen atoms.

"Pharmaceutically acceptable excipient " means an excipient that is useful in preparing a pharmaceutical composition that is generally safe, non-toxic, and desirable, and includes excipients that are acceptable for veterinary use as well as for human pharmaceutical use. Such excipients may be solid, liquid, semisolid, or, in the case of an aerosol composition, gaseous.

"Pharmaceutically acceptable salts" means salts that are pharmaceutically acceptable and have the desired pharmacological properties. Such salts include salts that may be formed where acidic protons present in the compounds are capable of reacting with inorganic or organic bases. Suitable inorganic salts include those formed with the alkali metals, e.g. sodium and potassium, magnesium, calcium, and aluminum. Suitable organic salts include those formed with organic bases such as the amine bases, e.g. ethanolamine, diethanolamine, triethanolamine, tromethamine, N-methylglucamine, and the like. Such salts also include acid addition salts formed with inorganic acids (e.g. hydrochloric and hydrobromic acids) and organic acids (e.g. acetic acid, citric acid, maleic acid, and the alkane- and arene-sulfonic acids such as methanesulfonic acid and benzenesulfonic acid). When there are two acidic groups present, a pharmaceutically acceptable salt may be a mono-acid-mono-salt or a di-salt; and similarly where there are more than two acidic groups present, some or all of such groups can be salified.

A "protecting group" has the meaning conventionally associated with it in organic synthesis, i.e. a group that selectively blocks one or more reactive sites in a multifunctional compound such that a chemical reaction can be carried out selectively on another unprotected reactive site and such that the group can readily be removed after the selective reaction is complete.

A "therapeutically effective amount" means the amount that, when administered to an animal for treating a disease, is sufficient to effect treatment for the disease.

"Treating" or "treatment" of the disease includes preventing the disease from occurring in a mammal that may be predisposed to the disease but does not yet experience or exhibit symptoms of the disease (prophylactic treatment), inhibiting the disease (slowing or arresting its development), providing relief from the symptoms or side-effects of the disease (including palliative treatment), and relieving the disease (causing regression of the disease). "Treating" amyloidosis includes any one or more of the following: preventing, inhibiting, reducing, disassembling, disrupting, and disaggregating amyloid fibrils and amyloid protein deposits, such as Aβ and the other amyloids referred to in the BACKGROUND TO THE INVENTION. "Treating" an α-synuclein fibril disease includes any one or more of the following: preventing, inhibiting, reducing, disassembling, disrupting, and disaggregating α-synuclein fibrils and α-synuclein-associated protein deposits, such as those in Lewy body disease and Parkinson's disease.

The compounds found in the compositions and used in the methods of this invention may possess one or more chiral centers, and can therefore be produced as individual stereoisomers or as mixtures of stereoisomers, depending on whether individual stereoisomers or mixtures of stereoisomers of the starting materials are used. Unless indicated otherwise, the description or naming of a compound or group of compounds is intended to include both the individual stereoisomers or mixtures (racemic or otherwise) of stereoisomers. Methods for the determination of stereochemistry and the separation of stereoisomers are well known to a person of ordinary skill in the art [*see* the discussion in Chapter 4 of March J: Advanced Organic Chemistry, 4th ed. John Wiley and Sons, New York, NY, 1992].

### Presently Preferred Compounds

While the broadest definition of the invention is set out in the Summary of the Invention, certain compounds of this invention are presently preferred.

Presently preferred compounds of this invention are compounds where:
R₁ and R₂ are independently selected from the group consisting of hydrogen; C₁₋₆ alkyl, C₁₋₆ alkoxy, and C₁₋₆ alkylthio (in each of which the alkyl group is optionally substituted with 1 to 5 halogen atoms); and halo;
X is selected from hydrogen and the group consisting of C₁₋₂₂ alkyl, each optionally substituted with 1 to 5 moieties selected from the group consisting of hydroxy, and amino, nitro, C₁₋₆ alkoxy, C₁₋₆ alkylthio, and C₁₋₆ alkylcarboxyl,
and the pharmaceutically acceptable salts thereof.

Preferred compounds include the compounds of formula A and formula B.

A number of different preferences have been given above, and following any one of these preferences results in a compound or the composition or method of this invention that is more presently preferred than a compound in which that particular preference is not followed. However, these preferences are generally independent and additive; and following more than one of these preferences may result in a more presently preferred compound than one in which fewer of the preferences are followed.

Presently preferred compounds of this invention include 1,2,4-benzenetriol, 5-hydroxydopamine.

### Pharmacology and Utility

The compounds of this invention act to inhibit or prevent amyloid fibril formation, inhibit or prevent amyloid fibril growth, and/or cause disassembly, disruption, and/or disaggregation of preformed amyloid fibrils and amyloid protein deposits. Their activity can be measured *in vitro* by methods such as those discussed in Examples 1 through 4 and Assay 1 below, while their activity *in vivo* against amyloidoses can be measured in animal models, such as those of Alzheimer's disease and in humans by a method such as that discussed in Assay 2 below.

The compounds of this invention also act to inhibit or prevent α-synuclein fibril formation, inhibit or prevent α-synuclein fibril growth, and/or cause disassembly, disruption, and/or disaggregation of preformed α-synuclein fibrils and α-synuclein-associated protein deposits. Their activity can be measured *in vitro* by methods similar to those discussed in Examples 1 through 4 below.

The therapeutic ratio of a compound can be determined, for example, by comparing the dose that gives effective anti-fibril (anti-amyloid or anti-α-synuclein activity in a suitable *in vivo* model in a suitable animal species such as the mouse, with the dose that gives significant weight loss (or other observable side-effects) in the test animal species.

### Pharmaceutical compositions and administration

In general, compounds of this invention will be administered in pure isolated form in therapeutically effective amounts by any of the usual modes known in the art, either singly or in combination with at least one other compound of this invention and/or at least one other conventional therapeutic agent for the disease being treated. A therapeutically effective amount may vary widely depending on the disease, its severity, the age and relative health of the animal being treated, the potency of the compound(s), and other factors. As anti-fibril agents, therapeutically effective amounts of compounds of this invention may range from 1-1000 mg/Kg body weight; for example, 10-100 mg/Kg. A person of ordinary skill in the art will be able without undue experimentation, having regard to that skill and this disclosure, to determine a therapeutically effective amount of a compound of this invention for the treatment of amyloidosis.

In general, compounds of this invention will be administered as pharmaceutical compositions by one of the following routes: oral, topical, systemic (e.g. transdermal, intranasal, or by suppository), or parenteral (e.g. intramuscular, subcutaneous, or intravenous injection). Compositions may take the form of tablets, pills, capsules, semisolids, powders, sustained release formulations, solutions, suspensions, elixirs, aerosols, or any other appropriate compositions; and comprise at least one compound of this invention in combination with at least one pharmaceutically acceptable excipient. Suitable excipients are well known to persons of ordinary skill in the art, and they, and the methods of formulating the compositions, may be found in such standard references as Alfonso AR: Remington's Pharmaceutical Sciences, 17th ed., Mack Publishing Company, Easton PA, 1985. Suitable liquid carriers, especially for injectable solutions, include water, aqueous saline solution, aqueous dextrose solution, and glycols.

In particular, the compound(s) - preferably only one such compound is administered in any particular dosage form - can be administered, orally, for example, as tablets, troches, lozenges, aqueous or oily suspension, dispersible powders or granules, emulsions, hard or soft capsules, or syrups or elixirs. Compositions intended for oral use may be prepared according to any method known in the art for the manufacture of pharmaceutical compositions and such compositions may contain one or more agents selected from the group consisting of sweetening agents, flavoring agents, coloring agents and preserving agents in order to provide pharmaceutically elegant and palatable preparations.

Tablets contain the compound in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, maize starch or alginic acid; binding agents, for example, maize starch, gelatin or acacia, and lubricating agents, for example, magnesium stearate or stearic acid or tale. The tablets may be uncoated or they may be coated by known techniques to delay disintegration and absorption in the gastrointestinal tract and thereby provide a sustained action over a longer period. For example, a time delay material such as glycerol monostearate or glycerol distearate may be employed. Formulations for oral use may also be presented as hard gelatin capsules wherein the compound is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Aqueous suspensions contain the compound in admixture with excipients suitable for the manufacture of aqueous suspensions. Such excipients are suspending agents, for example, sodium carboxymethylcellulose, methylcellulose, hydroxypropylmethyl cellulose, sodium alginate, polyvinylpyrrolidone, gum tragacanth and gum acacia; dispersing or wetting agents may be naturally occurring phosphatides, for example lecithin, or condensation products of an alkylene oxide with fatty acids, for example polyoxyethylene stearate, or condensation products of ethylene oxide with long chain aliphatic alcohols, for example, heptadecaethyleneoxycetanol, or condensation products of ethylene oxide with partial esters derived from fatty acids such as hexitol such as polyoxyethylene sorbitol monooleate, or condensation products of ethylene oxide with partial esters from fatty acids and a hexitol annhydrides, for example, polyethylene sorbitan monooleate. The aqueous suspensions may also contain one or more preservatives, for example, ethyl or n-propyl p-hydroxybenzoate, one or more coloring agents, one or more flavoring agents, or one or more sweetening agents, such as sucrose or saccharin.

Oily suspensions may be formulated by suspending the compound in a vegetable oil, for example arachis oil, olive oil, sesame oil, or coconut oil or in a mineral oil such as liquid paraffin. The oily suspensions may contain a thickening agent, for example beeswax, hard paraffin or cetyl alcohol. Sweetening agents, such as those set forth below, and flavoring agents may be added to provide a palatable oral preparation. These compositions may be preserved by the addition of an antioxidant such as ascorbic acid. Dispersible powders and granules suitable for preparation of an aqueous suspension by the addition of water provide the active ingredient in admixture with a dispersing or wetting agent, a suspending agent and one or more preservatives. Suitable dispersing or wetting agents and suspending agents are exemplified by those already described above. Additional excipients, for example sweetening, flavoring and agents, may also be present.

The compounds may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally occurring phosphatides, for example soy bean, lecithin, and occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsion may also contain sweetening and flavoring agents. Syrups and elixirs may be formulated with sweetening agents, for example, glycerol, sorbitol or sucrose. Such formulations may also contain a demulcent, a preservative and flavoring and coloring agents.

The compound can also be administered by injection or infusion, either subcutaneously or intravenously, or intramuscularly, or intrasternally, or intranasally, or by infusion techniques in the form of sterile injectable or oleaginous suspension. The compound may be in the form of a sterile injectable aqueous or oleaginous suspensions. These suspensions may be formulated according to the known art using suitable dispersing of wetting agents and suspending agents which have been described above. The sterile injectable preparation may also be a sterile injectable solution or suspension in a non-toxic parenterally-acceptable diluent or solvent for example, as a solution in 1,3-butanediol. Among the acceptable vehicles and solvents that may be employed are water, Ringer's solution and isotonic sodium chloride solution. In addition, sterile, fixed oils are conventionally employed as a solvent or suspending tedium. For this purpose any bland fixed oils may be conventionally employed including synthetic mono- or diglycerides. In addition fatty acids such as oleic acid find use in the preparation of injectables.

Dosage regimens can be adjusted to provide the optimum therapeutic response. For example, several divided dosages may be administered daily or the dosage may be proportionally reduced as indicated by the exigencies of the therapeutic situation.

It is especially advantageous to formulate the compounds in dosage unit form for ease of administration and uniformity of dosage. Dosage unit form as used herein refers to physically discrete units suited as unitary dosages for the subjects to be treated; each containing a therapeutically effective quantity of the compound and at least one pharmaceutical excipient. A drug product will comprise a dosage unit form within a container that is labeled or accompanied by a label indicating the intended method of treatment, such as the treatment of an amyloid disease, such as Alzheimer's disease, or of a disease associated with α-synuclein fibril formation, such as Parkinson's disease. A "therapeutically effective dosage" preferably inhibits amyloidosis or a disease associated with α-synuclein fibril formation in a patient by at least 20, more preferably by at least 40%, even more preferably by at least 60%, and still more preferably by at least 80%, relative to untreated subjects.

### Preparation of the Compounds of this Invention

Many of the compounds used in the compositions and methods of this invention are well known to the art. They may be briefly described in such references as the Merck Index, 12the edition, Merck & Co., Inc., Whitehouse Station, New Jersey, 1996 (which typically provides a reference to a synthesis or isolation), and may be found in chemical catalogs, such as those of commercial suppliers such as Aldrich Chemical Company (Milwaukee, WI), Bachem (Torrance, CA), Sigma (St. Louis, MO).

For those compounds that are novel, the starting materials and reagents used in preparing these compounds are generally available from commercial suppliers such as Aldrich Chemical Company, Bachem, and Sigma, or are prepared by methods well known to a person of ordinary skill in the art following procedures described in such references as Fieser and Fieser's Reagents for Organic Synthesis, vols 1-17, John Wiley and Sons, New York, NY, 1991; Rodd's Chemistry of Carbon Compounds, vols. 1-5 and supps, Elsevier Science Publishers, 1989; Organic Reactions, vols 1-40, John Wiley and Sons, New York, NY, 1991; March J: Advanced Organic Chemistry, 4th ed. John Wiley and Sons, New York, NY, 1992; and Larock: Comprehensive Organic Transformations, VCH Publishers, 1989, and the syntheses of the novel compounds will be readily suggested to a person or ordinary skill in the art by reference to known analogs (such as the commercially available analogs referred to above) of the novel compounds. Many such preparations will involve the use of protecting groups, especially for the protection of the hydroxy groups that form an essential part of the compounds; and the knowledge and use of such protecting groups will be within the knowledge of a person of ordinary skill in the art.

The starting materials, intermediates, and compounds of this invention may be isolated and purified using conventional techniques, including filtration, distillation, crystallization, chromatography, and the like. They may be characterized using conventional methods, including physical constants and spectral data.

### Examples

The following non-limiting examples illustrate the invention.

### Example 1. Disassembly/disruption of Alzheimer's disease Aβ 1-42 fibrils by polyhydroxylated aromatic compounds

In this study, different types of commercially available compounds which consist of various polyhydroxylated aromatic containing structures were tested for their ability to cause a disassembly/disruption of pre-formed Alzheimer's disease amyloid fibrils containing Aβ 1-42. This type of activity would be important for any potential anti-amyloid drug which can be used in patients who already have substantial amyloid deposition in organs and/or tissues. For example, Alzheimer's disease patients in mid-to-late stage disease have abundant Aβ-containing amyloid deposits in their brains as part of both neuritic plaques and cerebrovascular amyloid deposits. A compound capable of causing disassembly/disruption of pre-existing amyloid deposits would be advantageous for use in these patients who are at latter stages of the disease process.

For the first study, 1 mg of Aβ 1-42 (Bachem Inc., Torrance, CA, USA) was dissolved in 1.0 ml of double distilled water (1 mg/ml solution). 25 µM of Aβ 1-42 was then incubated overnight (∼18 hours) at 37°C, in the absence or presence of 100µg/ml of the following compounds: 1) EDTA (Sigma Chemical Company, St. Louis, MO, USA), 2) myricetin (Acros, Somerville, New Jersey, USA), 3) exifone (Acros) 4) pyrogallol (Sigma), 5) tannic acid (Acros), 6) pyrocatechol (Acros), 7) quercetin (Sigma), 8) ellagic acid (Acros), 9) 1,2,4-benzenetriol (Acros), 10) 5-hydroxydopamine (Acros), 11) gallamide hydrate (Acros), 12) gallic acid (Sigma), 13) ethyl gallate (Acros), 14) quinic acid (Acros), 15) propyl gallate (Sigma), and 16) phloroglucide (Acros), each in the presence of 150 mM Tris HCl, 10 mM NaCl (pH 7.0) with 0.02% sodium azide. In this study, the Aβ 1-42:compound weight ratio was 1:1.

For the second study, 1 mg of Aβ 1-42 (Bachem) was dissolved in 1.0 ml of double distilled water (1 mg/ml solution). 25 µM of Aβ 1-42 was then incubated overnight (∼18 hours) at 37°C, in the absence or presence of 50µg/ml of the following compounds: 1) gallic acid, 2) ethyl gallate, 3) quinic acid, 4) gallamide trihydrate, 5) ellagic acid, 6) propyl gallate, and 7) pyrogallol, each in the presence of 150 mM Tris HCl, 10 mM NaCl (pH 7.0) with 0.02% sodium azide . In this study, the Aβ 1-42:compound weight ratio was 2:1.

A previously described method of measuring amyloid fibril formation utilizing Thioflavin T fluorometry (H Naiki et al., Lab. Invest. 65:104-110, 1991; H Levine III, Protein Sci. 2:404-410, 1993; H Levine III, Amyloid: Int. J. Exp. Clin. Invest. 2:1-6, 1995; H Naiki and K. Nakakuki, Lab. Invest. 74:374-383, 1996) was employed to identify potential therapeutic compounds capable of causing a disassembly/disruption of Alzheimer's Aβ 1-42 amyloid fibrils. Thioflavin T is known to bind to fibrillar amyloid proteins, and an increase in fluorescence correlates with an increase in amyloid fibril formation, whereas a decrease in fluorescence correlates with a decrease in amyloid fibrils due to disassembly and/or disruption. The Alzheimer's Aβ protein (1-42) when placed in solution, such as distilled water, tends to spontaneously form amyloid fibrils. Using this sensitive assay, any decreases or increases in fluorescence was previously shown to correlate with a decrease or increase in the amount of amyloid fibrils (see the documents cited above), allowing one to identify and quantitate the extent of potential inhibitors and/or enhancers of Alzheimer's Aβ 1-42 amyloid fibrils.

To assess the effects of each compound on potential disassembly/ disruption of preformed Aβ 1-42 fibrils, 50 µl of Aβ 1-42 with or without test compounds (described above) were added to 1.2 ml of 100µM Thioflavin T (Sigma) in 50mM NaH₂PO₄ (pH 6.0) for fluorometry readings. Studies indicated that increasing concentrations of Aβ gave a proportional increase in fluorescence in the presence of 100µM Thioflavin T, ruling out the presence of any disproportionate inner filter effects in these studies. Fluorescence emission at 482 nm was measured on a Turner instrument-model 450 fluorometer at an excitation wavelength of 450 nm. For each determination, the fluorometer was calibrated by zeroing in the presence of the Thioflavin T reagent alone, and by setting the 50 ng/ml riboflavin (Sigma Chemical Co., St. Louis, MO) in the Thioflavin T reagent to 1800 fluorescence units. All fluorescence determinations were based on these references and any fluorescence given off by any of the compounds in the presence of the Thioflavin T reagent was always subtracted from all pertinent readings.

For all fibrillogenesis studies utilizing Thioflavin T fluorometry, as disclosed herein, comparisons of amyloid protein in the presence or absence of test compounds were based on paired Student's *t* tests with data shown as the mean of triplicate measurements ± standard deviation.

As shown in Table 1, the polyhydroxylated aromatic compounds caused a disassembly/disruption of Aβ 1-42 amyloid fibril as determined by inhibition of Thioflavin T fluorescence. All results were significant at the p < 0.005 level, except that for quinic acid at the 2:1 ratio (asterisked in Table 1), which was not significant.

**Table 1: Disassembly/disruption of Alzheimer's 1-42 fibrils, as indicated by Thioflavin T fluorescence inhibition**

| | Fluorescence inhibition, %, at the Aβ 1-42:compound w/w ratios given | |
|---|---|---|
| Compound name | 1:1 | 2:1 |
| Myricetin | 94 ± 0.9 | |
| Exifone | 93 ± 1.4 | |
| Pyrogallol | 89 ± 6.7 | 72 ± 3.8 |
| Tannic acid | 77 ± 1.3 | |
| Pyrocatechol | 77 ± 2.6 | |
| Quercetin | 76 ± 0.6 | |
| Ellagic acid | 74 ± 1.4 | 62 ± 3.9 |
| 1,2,4-Benzenetriol | 71 ± 3.3 | |
| 5-Hydroxydopamine | 70 ± 1.1 | |
| Gallamide trihydrate | 65 ± 12.3 | 60 ± 2.2 |
| Gallic acid | 57 ± 1.9 | 44 ± 1.7 |
| Ethyl gallate | 49 ± 0.8 | 30 ± 3.7 |
| Quinic acid | 31 ± 9.0 | 0.5 ± 3.9* |
| Phloroglucide | 30 ± 0.6 | |
| Propyl gallate | 29 ± 2.8 | 38 ± 4.8 |

EDTA, a known chelating agent, caused no significant disassembly/disruption of Aβ 1-42 amyloid fibrils, suggesting that the inhibitory effects observed with polyhydroxylated aromatic compounds was not attributable to their ability to complex metals.

### Example 2. Dose-dependent disassembly/disruption of Alzheimer's disease Aβ 140 fibrils by tannic acid and gallic aid

In this study, the potential dose-dependent effects of tannic acid and gallic acid on disassembly/ disruption of pre-formed Aβ 1-40 was assessed. In this experiment, 1 mg of Aβ 1-40 (Bachem Inc., Torrance, CA, USA; Lot # T-20824) was dissolved in 1.0 ml of double distilled water (1 mg/ml solution) and incubated for 4 days at 37°C to spontaneously induce fibril formation. 25 µM of pre-fibrillized Aβ 1-40 was then incubated overnight (∼18 hours) at 37°C, in the absence or presence of increasing amounts (25µg/ml, 50µg/ml, 75µg/ml and 100µg/ml) of tannic acid or gallic acid (each in the presence of 150 mM Tris HCl, 10 mM NaCl, pH 7.0, with 0.02% sodium azide). The Aβ:compound weight ratios were therefore 4:1, 2:1, 4:3, and 1:1, respectively. 50 µl aliquots were then added to 1.2 ml of 100µM Thioflavin T (Sigma) in 50mM NaH₂PO₄ (pH 6.0) for fluorometry readings as described in Example 1 above.

As shown in Table 2, both tannic acid and gallic acid caused a dose-dependent disassembly/disruption of Aβ 1-40 amyloid fibrils as indicated by a dose-deperident inhibition of Thioflavin T fluorescence. All results were significant at the p < 0.005 level, except that for gallic acid at the 4:1 ratio (asterisked in Table 2), which was significant at the p < 0.05 level.

**Table 2: Dose-dependent disassembly/disruption of Alzheimer's 1-40 fibrils, as indicated by Thioflavin T fluorescence inhibition**

| | Fluorescence inhibition, %, at the Aβ 1-40:compound w/wratios given | | | |
|---|---|---|---|---|
| Compound name | 4:1 | 2:1 | 4:3 | 1:1 |
| Tannic acid | 31 ± 4.8 | 42 ± 2.8 | 49 ± 3.7 | 53 ± 4.2 |
| Gallic acid | 14 ± 8.2* | 22 ± 3.3 | 34 ± 3.6 | 45 ± 4.1 |

### Example 3. Disaggregation of Alzheimer's disease Aβ 1-40 fibrils by polyhydroxylated aromatic compounds

In this study, a Congo red-Aβ spectrophotometric assay (Klunk et al., Anal. Biochem. 266:66-76, 1999) was modified to determine the effectiveness of polyhydroxylated aromatic compounds on the disaggregation of pre-formed Aβ 1-40 amyloid fibrils. For this assay, 1 mg of Aβ 1-40 (Bachem) was incubated for 4 days in distilled water at 37°C to spontaneously produce amyloid fibrils. 25µM of fibrillized Aβ 1-40 was then incubated in triplicate with various test compounds for 3 days at 37°C in Tris-buffered saline (TBS)(100 mM Tris; 50 mM NaCl; pH 7.0, with 0.02% sodium azide), at an Aβ:compound weight ratio of 2:1. Following incubation, 50µl of 360 µM Congo red (Sigma) in distilled water was then added to 250 µl of each incubation mixture, giving a final Aβ:Congo red molar ratio of 1:3. After 10 minutes, the absorbance at 405 nm (reference wavelength to account for the absorbance of Congo red alone at 540 nm) and 540 nm (sample absorbance where "sample" refers to Aβ alone, test compound alone, or Aβ plus test compound, all in the presence of Congo red) was determined using a Biorad Model 550 ELISA Plate Reader (Biorad, Hercules, CA, USA). The absorbance at wavelength 405 nm was automatically subtracted by the ELISA plate reader from the absorbance at wavelength 540 nm (difference is referred to as Δ absorbance)(see Klunk et al. cited above). Therefore, the Δ absorbance reading at 540 nm was proportional to the amount of aggregated Aβ left in solution (Klunk et al.).

For all experiments involving test compounds, the Δ absorbance reading at 540 nm of the test compound alone (in the absence of Aβ), was always subtracted from the corresponding Δ absorbance reading at 540 nm of the test compound in the presence of Aβ. Using this modification of the method of Klunk et al., the use of a greater final concentration of Congo red, i.e. 60µM instead of 14µM, in the presence of fibrillar Aβ gave an overall absorbance at 540 nm that was always below 1.0 Absorbance Unit (AU), and well within the linear absorbance range.

The following polyhydroxylated aromatic containing compounds were tested using the above described Congo red-Aβ spectrophotometric assay to determine their effectiveness on disaggregation of pre-formed Aβ 1-40 amyloid fibrils: 1) gallic acid, 2) ethyl gallate, 3) quinic acid, 4) gallamide trihydrate, 5) ellagic acid, 6) propyl gallate, and 7) pyrogallol.

The polyhydroxylated aromatic compounds had varying effects on causing disaggregation of pre-aggregated Aβ 1-40 amyloid fibrils as determined using the Congo red spectrophotometric assay described above. The results were significant at the p < 0.005 level, except for propyl gallate (asterisked in Table 3) at the p < 0.05 level, and quinic acid (double asterisked), which was not significant.

**Table 3: Disaggregation of Alzheimer's 1-40 fibrils, as indicated by Congo red spectrophotometry**

| Compound name | Decrease in absorbance, % |
|---|---|
| Gallic acid | 52 ±0.4 |
| Ethyl gallate | 28 ± 5.0 |
| Quinic acid | 0 + 5.0** |
| Gallamide trihydrate | 31 ± 1.9 |
| Ellagic acid | 54 ± 2.8 |
| Propyl gallate | 17 ± 7.3* |
| Pyrogallol | 63 ± 3.4 |

### Example 4. Dose-dependent disaggregation of Alzheimer's disease Aβ 1-40 fibrils by tannic acid and gallic acid

In this study, the potential dose-dependent effects of tannic acid and gallic acid on the disaggregation of fibrillized Aβ 1-40 was assessed. In this experiment, the modified Congo red-Aβ spectrophotometric assay (Klunk et al, Anal. Biochem. 266:66-76, 1999) was used as described above (i.e. Example 4). However, in this specific experiment increasing amounts of tannic acid or gallic acid (i.e. 25µg/ml, 50µg/ml, 75µg/ml and 100µg/ml) were tested following an overnight (∼18 hours) incubation at 37°C in the presence of 25µM of Aβ 1-40 (Bachem).

As shown in Table 4, both tannic acid and gallic acid caused a dose-dependent disaggregation of Aβ 1-40 amyloid fibril as determined by decreases in Thioflavin T fluorescence. All results were significant at the p < 0.001 level, except that for gallic acid at the 4:1 ratio (asterisked in Table 4), which was significant at the p < 0.05 level.

**Table 4: Dose-dependent disaggregation of Alzheimer's 1-40 fibrils, as indicated by Congo red spectrophotometry**

| | Decrease in absorbance, %, at the Aβ 1-42: compound w/w ratios given | | | |
|---|---|---|---|---|
| Compound name | 4:1 | 2:1 | 4:3 | 1:1 |
| Tannic acid | 42 ± 5.2 | 48 ± 6.8 | 59 ± 6.4 | 61 ± 11.1 |
| Gallic acid | 17 ± 9.5* | 22 ± 4.0 | 30 ± 6.0 | 32 ± 4.8 |

### Example 5. Disassembly/disruption of islet amyloid fibrils (amylin) by polyhydroxylated aromatic compounds

90% of patients with type II diabetes demonstrate the deposition and accumulation of amyloid fibrils in the islets of Langerhans in the pancreas (Cooper et al., Proc. Natl. Acad. Sci. USA 84:8628-8632, 1987). This amyloid protein involved consists of a 37 amino acid protein known as islet amyloid polypeptide or amylin. Islet amyloid is believed to contribute to the destruction of the beta-cells of the pancreas, thus eventually leading many patients to become insulin-dependent (i.e. type I diabetes). Amylin has the ability to also form substantial amyloid fibrils immediately when placed in solution. The next study was therefore implemented to determine whether some of the specific polyhydroxylated aromatic containing compounds which cause a disassembly/disruption of Aβ fibrils, also cause a disassembly/disruption of islet amyloid fibrils.

For this study, the method of Thioflavin T fluorometry as described in Example 1 was used. Briefly, 25 µM of human amylin (Bachem) was incubated overnight (∼18 hours) at 37°C, alone or in the presence of 100µg/ml of the following compounds: 1) exifone, 2) myricetin, and 3) tannic acid, each in the presence of 150 mM Tris HCl, 10 mM NaCl, pH 7.0, with 0.02% sodium azide, at an amylin:compound weight ratio of 1:1.

Following Thioflavin T fluorometry readings as described in Example 1, 5µl aliquots of amylin only, amylin + Myricetin, amylin + exifone, and amylin + tannic acid were also taken, allowed to air dry overnight on gelatin-coated slides, and stained with Congo red as previously described (Castillo et al., Diabetes 47:612-620, 1998).

As shown in Table 5, the polyhydroxylated aromatic compounds which were very effective in causing a disassembly/disruption of Aβ 1-42 amyloid fibrils were also effective in causing a disassembly/disruption of islet amyloid fibrils. All results were significant at the p < 0.005 level.

**Table 5: Disassembly/disruption of amylin fibrils, as indicated by Thioflavin T fluorescence inhibition**

| Compound name | Fluorescence inhibition, % |
|---|---|
| Myricetin | 97.4 ± 0.3 |
| Exifone | 99.1 ± 0.5 |
| Tannic acid | 83.8 ± 1.4 |

Congo red staining experiments confirmed the disassembly/disruption of amylin fibrils by polyhydroxylated aromatic compounds initially demonstrated by Thioflavin T fluorometry studies as described above. Congo red staining of amylin alone demonstrated positive staining (i.e. classic red/green birefringence as viewed under polarized light and indicative of amyloid) (Puchtler et al., J. Histochem. Cytochem. 10:355-364, 1962). In comparison, an overnight incubation with exifone, myricetin or tannic acid resulted in a marked decrease in Congo red staining, suggestive of an amylin fibril disassembly/disruption.

Further in vitro and in vivo assays may be used to test the compounds for their effectiveness in the treatment of Alzheimer's disease, such as those described in European Published Patent Application No. 0 659 418.

Stock solutions of peptides (1 mM) are freshly prepared in pyrogen-free sterile water and diluted to the indicated concentrations in defined culture media. Rat hippocampal cultures (10-14 days *in vitro*) are treated with peptides or vehicle for four days. The viability of the rat cortical cultures is visually assessed by phase contrast microscopy and quantified by measuring lactate dehydrogenase (LDH) released into the culture media.

### Assay 1

Primary rat hippocampal neurons are cultured *in vitro* with standard cell culture techniques. Amyloid-beta (Aβ) peptide is added to cultured cells at a normally toxic concentration of 25-50 µM. After 4 days of treatment, viability is assessed by measurement of lactate dehydrogenase (LDH) released into culture medium. Lactate dehydrogenase (LDH) is measured in 20 µl aliquots of conditioned defined DMEM using a standard 340 nm kinetic LDH assay (Sigma Catalog Number #228-20) in a 96 well format. Assays are performed at 37°C in a PC-driven EL340 Microplate Biokinetics plate reader (Bio-Tek Instruments) using Delta Soft II software (v. 3.30B, BioMetallics, Inc.) for data analysis. Quality control standards containing normal and elevated levels of serum LDH (for example, Sigma Enzyme Controls 2N and 2E) are run with every assay. Results are expressed as units of LDH/L where 1 unit is defined as the amount of enzyme that will catalyze the formation of 1 micromole of nicotinamide adenine dinucleotide per minute under conditions of the assay. For protection studies, a compound of formula 1 is added to cultures prior to and/or concurrently with the amyloid- beta treatment.

Activity of the compounds is illustrated by a decrease in LDH released into the media (a neurotoxic indicator), as compared to control.

### Assay 2

Five to fifty women are selected for a clinical study. The women are postmenopausal, i.e., have ceased menstruating for between 6 and 12 months prior to the study's initiation, have been diagnosed with early stage Alzheimer's disease (AD), are expected to have worsening symptoms of AD within the study period, but are in good general health otherwise. The study has a placebo control group, i.e., the women are divided into two groups, one of which receives the compound of this invention and the other receives a placebo. The patients are benchmarked as to memory, cognition, reasoning, and other symptoms associated with AD. Women in the test group receive a therapeutic dose of the compound per day by the oral route. They continue this therapy for 6-36 months. Accurate records are kept as to the benchmarked symptoms in both groups and at the end of the study these results are compared. The results are compared both between members of each group and also the results for each patient are compared to the symptoms reported by each patient before the study began. Activity of the compound is illustrated by an attenuation of the typical cognitive decline and/or behavioral disruptions associated with AD.

Utility of the compounds is evidenced by activity in at least one of the above assays.

While this invention has been described in conjunction with specific embodiments and examples, it will be apparent to a person of ordinary skill in the art, having regard to this disclosure, that equivalents of the specifically disclosed materials and techniques will also be applicable to this invention; and such equivalents are intended to be included within the following claims.

## Claims

1. Use of a pharmaceutical composition in the manufacture of a medicament for treating a mammal suffering from amyloidosis, comprising a therapeutically effective amount of an isolated pure compound selected from the compounds of formula A and formula B: 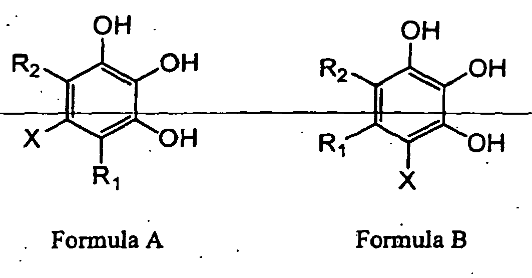 where
R₁ and R₂ are independently selected from hydrogen and non-interfering substituents;
X is selected from hydrogen and the group consisting of
(a) amino, C₁₋₆ alkylamino, di(C₁₋₆ alkyl)amino, and cycloamino,
(b) C₁₋₂₂ alkyl,
and the group of compounds consisting of pyrogallol, pyrocatechol, 5-hydroxydopamine 1,2,4-benzenetriol, carbidopa, deoxyepinephrine, dioxethedrine, dopa, dopamine, droxidopa,
and the pharmaceutically acceptable salts thereof.

2. The use of Claim 1 where R₁ and R₂ are independently selected from the group consisting of hydrogen; C₁₋₆ alkyl, C₁-₆ alkoxy, and C₁₋₆ alkylthio (in each of which the alkyl group is optionally substituted with 1 to 5 halogen atoms); and halo.

3. The use of Claim 1, where the compound of formula A or formula B, or a pharmaceutically acceptable salt thereof is selected from the group consisting of 5-hydroxydopamine and the pharmaceutically acceptable salts thereof.

4. The use of any one of Claims 1 to 3 where the amyloidosis is selected from the group of diseases consisting of Alzheimer's disease, Down's syndrome, hereditary cerebral hemorrhage with amyloidosis of the Dutch type, the amyloidosis of chronic inflammation, the amyloidosis of malignancy and familial Mediterranean fever, the amyloidosis of multiple myeloma and B-cell dyscrasias, the amyloidosis of type II diabetes, the amyloidosis of the prion diseases, Creutzfeldt-Jakob disease, Gerstmann-Straussler syndrome, kuru, scrapie, the amyloidosis associated with long-term hemodialysis, the amyloidosis associated with carpal tunnel syndrome, senile cardiac amyloidosis, familial amyloidotic polyneuropathy, and the amyloidosis associated with endocrine tumors.

5. The use of Claim 4 where the amyloidosis is Alzheimer's disease.

## Patentansprüche

1. Verwendung einer pharmazeutischen Zusammensetzung zur Herstellung eines Arzneimittels zur Behandlung eines Säugetieres, das unter Amyloidose leidet, umfassend eine therapeutisch wirksame Menge einer isolierten reinen Verbindung, ausgewählt aus der Gruppe bestehend aus Formel A und Formel B: 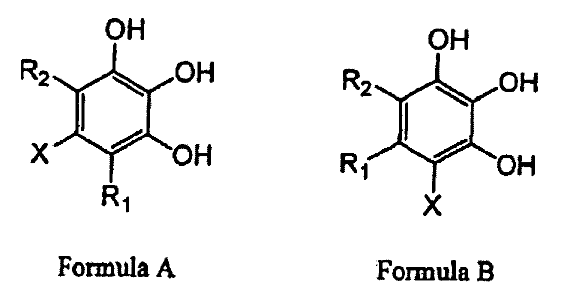 wobei
R₁ und R₂ unabhängig voneinander ausgewählt sind aus Wasserstoff und nicht-interferierenden Substituenten;
X ausgewählt ist aus Wasserstoff und der Gruppe bestehend aus
(a) Amino, C₁₋₆ Alkylamino, di(C₁₋₆-Alkyl)amino, und Cycloamino,
(b) C₁₋₂₂ -Alkyl
und der Gruppe Verbindungen bestehend aus Pyrogallol, Pyrocatechol, 5-Hydoxydopamin, 1,2,4-Benzentriol, Carbidopa, Desoxyepinephrin, Dioxethedrin, Dopa, Dopamin, Droxidopa,
und deren pharmazeutisch annehmbaren Salzen.

2. Die Verwendung nach Anspruch 1, wobei R₁ und R₂ unabhängig voneinander ausgewählt sind aus der Gruppe bestehend aus Wasserstoff, C₁₋₆-Alkyl, C₁₋₆-Alkoxy, und C₁₋₆-Alkylthio (wobei jede der Alkylgruppen wahlweise mit 1 bis 5 Hologenatomen substituiert ist); und einer Halo-Gruppe.

3. Die Verwendung nach Anspruch 1, wobei die Verbindung nach Formel A oder Formel B, oder ein pharmazeutisch annehmbares Salz davon, ausgewählt ist aus der Gruppe bestehend aus 5-Hydroxydopamin und dessen pharmazeutisch annehmbaren Salzen.

4. Die Verwendung nach einem der Ansprüche 1 bis 3, wobei die Amyloidose ausgewählt ist aus der Gruppe von Krankheiten bestehend aus Morbus Alzheimer, Down-Syndrom, erblicher cerebraler Hämorrhagie mit Amyloidose-Dutch-Typ (HCHWA-D), Amyloidose von chronischer Entzündung, Amyloidose von Krebs und familiärer paroxysmaler Peritonitis ("familial Mediterranean fever"), Amyloidose von multiplem Myelom und B-Zell-Dyskrasie, Amyloidose von Typ 11- Diabetes, Amyloidose von Prionenerkrankungen, Creutzfeldt-Jakob-Krankheit, Gerstmann-Straussler-Syndrom, Kuru, Scrapie, Amyloidose die mit Langzeithämodialyse einhergeht, Amyloidose die mit dem Carpaltunnelsyndrom einhergeht, senile Herzamyloidose,familiäre Amyloid-Polyneuropathie, und Amyloidose die mit endokrinen Tumoren einhergeht.

5. Die Verwendung nach Anspruch 4, wobei die Amyloidose Morbus Alzheimer ist.

## Revendications

1. Utilisation d'une composition pharmaceutique pour la fabrication d'un médicament pour traiter un mammifère souffrant d'amylose, comprenant une quantité efficace au plan thérapeutique d'un composé pur isolé choisi parmi les composés de formule A et de formule B : 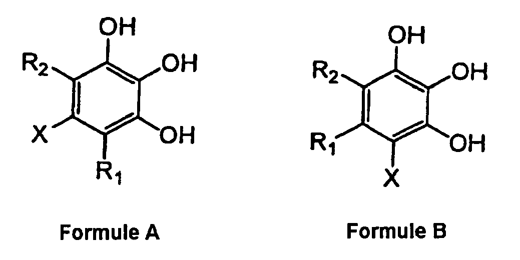 dans lesquelles R₁ et R₂ sont choisis indépendamment parmi l'hydrogène et des substituants non interférants ;
X est choisi parmi l'hydrogène et dans le groupe constitué par
(a) amino, C ₁₋₆ alkylamino, di(C ₁₋₆ alkyl) amino et cycloamino,
(b) C ₁₋₂₂ alkyl;
et le groupe de composés constitué par le pyrogallol, le pyrocatéchol, la 5-hydroxydopamine, le 1,2,4-benzènetriol, la carbidopa, la désoxyépinéphrine, la dioxéthédrine, la dopa, la dopamine, la droxidopa et les sels pharmaceutiquement acceptables de ceux-ci.

2. Utilisation selon la revendication 1, dans laquelle R₁ et R₂ sont choisis indépendamment dans le groupe constitué par l'hydrogène ; C ₁₋₆ alkyl, C ₁₋₆ alcoxy et C ₁₋₆ alkylthio (dans chacun desquels le groupe alkyle est éventuellement substitué par 1 à 5 atomes d'halogène) ; et un atome d'halogène.

3. Utilisation selon la revendication 1, dans laquelle le composé de formule A ou de formule B, ou un sel pharmaceutiquement acceptable de celui-ci est choisi dans le groupe constitué par la 5-hydroxydopamine et les sels pharmaceutiquement acceptables de celle-ci.

4. Utilisation selon l'une quelconque des revendications 1 à 3, dans laquelle l'amylose est choisie dans le groupe de maladies constitué par la maladie d'Alzheimer, le syndrome de Down, l'hémorragie cérébrale héréditaire avec une amylose du type Dutch, l'amylose d'une inflammation chronique, l'amylose d'une tumeur maligne et de la fièvre méditerranéenne familiale, l'amylose du myélome multiple et de la dyscrasie des cellules B, l'amylose du diabète de type II, l'amylose des maladies du prion, la maladie de Creutzfeldt-Jakob, le syndrome de Gerstmann-Straussler, le kuru, la tremblante du mouton, l'amylose associée à une hémodialyse à long terme, l'amylose associée au syndrome du tunnel carpien, l'amylose cardiaque sénile, la neuropathie multiple amyloïde familiale et l'amylose associée à des tumeurs endocrines.

5. Utilisation selon la revendication 4, dans laquelle l'amylose est la maladie d'Alzheimer.
